# FASCICULE DE BREVET EUROPEEN

(11) **EP 2 205 722 B1**
(45) Date de publication et mention de la délivrance du brevet: **26.12.2012**
(21) Numéro de dépôt: 08865596.4
(22) Date de dépôt: 06.10.2008
(51) Int. Cl.: C12N 5/02, A61K 38/05, A61K 35/32, A61P 19/02

(54) **MÉTHODE POUR STIMULER LA PROLIFÉRATION DE CELLULES DIFFÉRENCIÉES APPARTENANT AU LIGNAGE CHONDROGÉNIQUE**
VERFAHREN ZUR STIMULATION DER PROLIFERATION ZUR CHONDROGENEN LINIE GEHÖRENDER DIFFERENZIERTER ZELLEN
METHOD FOR STIMULATING THE PROLIFERATION OF DIFFERENTIATED CELLS BELONGING TO THE CHONDROGENIC LINEAGE

(30) Priorité: 10.10.2007 FR 0707113
(43) Date de publication de la demande: 14.07.2010
(73) Titulaire: MACO PHARMA, 59420 Mouvaux (FR)
(72) Inventeur: BRATOSIN, Daniela, R-40372 Bucuresti, Sector 4 (RO); BUIA TAKACS, Luminita, R-60903 Bucuresti (RO); MONTREUIL, Jean, F-59491 Villeneuve-d'Ascq cedex (FR); HERON, Antoine, F-67120 Ersgersheim (FR)
(74) Mandataire: Sayettat, Julien Christian
(86) Numéro de dépôt international: PCT/FR2008/001396
(87) Numéro de publication internationale: WO 2009/080914

(56) Documents cités:
- WO-A-99/48470
- WO-A-2005/084684
- US-A- 5 468 635

## Description

L'invention concerne une méthode de culture pour stimuler la prolifération de cellules différenciées appartenant au lignage chondrogénique, ainsi qu'une composition comprenant de telles cellules différenciées et un médicament pour le traitement local de pathologies liées à l'appareil ostéo-articulaire.

L'invention s'applique à la culture de cellules à visée thérapeutique, notamment la culture de chondrocytes en vue du traitement des lésions ostéoarthritiques, ainsi qu'aux médicaments destinés au traitement des lésions ostéoarthritiques.

La culture des chondrocytes est actuellement un enjeu important, notamment dans le cadre de la régénération tissulaire en médecine humaine et vétérinaire. En effet, l'autotransplantation de cellules cartilagineuses dans un tissu malade est une méthode avantageuse de traitement de pathologies du cartilage, comme par exemple l'ostéoarthrite.

En effet, afin d'éviter l'emploi d'une méthode intrusive de remplacement total des articulations ostéoarthritiques, des cellules de cartilage sont prélevées sur le patient puis cultivées in vitro dans un milieu d'expansion des chondrocytes, de manière à être multipliées et enfin réimplantées dans le tissu. La partie de l'articulation atteinte d'ostéoarthrite est ainsi reconstruite et greffée sur la partie saine.

Les milieux de culture des chondrocytes comportent en général un milieu de base de type DMEM, du sérum de veau foetal ou du sérum bovin foetal (FBS) et éventuellement d'autres éléments tels qu'un mélange d'antibiotiques. Le document US6,558,949 propose un milieu de culture amélioré pour chondrocytes comprenant des facteurs de croissance et dans lequel le FBS est remplacé par du sérum humain. Ce milieu permet d'augmenter la vitesse de prolifération des chondrocytes mais n'améliore pas le taux de prolifération de manière significative.

Le document WO 2005/084684 propose de cultiver les chondrocytes sur un substrat poreux en présence d'une composition comprenant a) de la lubricine, du protéoglycane 4 et/ou un phospholipide et b) de l'acide hyaluronique, du glycosaminoglycane et/ou des dérivés de ces substances. Cette composition est également utilisée in situ pour traiter un cartilage défectueux.

D'autres documents divulguent des utilisations particulières de la glycyglycine. Par exemple, le document WO 99/48470 décrit une composition dermato-cosmétique permettant de lutter contre le vieillissement cutané photo-induit et de stimuler le métabolisme cellulaire pour améliorer la qualité de la peau et des phanères. La composition contient de l'oléamide de glycylglycine et éventuellement un agent anti-glyquant comme l'aminoguanidine, la glycylglycine, le bromure de phémacylthiazolium, et/ou de l'acide oleique.

Le document US 5 468 635 décrit un milieu de culture sans sérum comprenant un lysozyme en tant que stimulateur de la croissance cellulaire. Ce milieu de culture comprend en outre un milieu de base, des acides aminés, des vitamines, d'autres composés organiques, des hormones, des métaux et des tampons, à savoir la glycylglycine et l'hydrogénocarbonate de sodium.

Des méthodes d'amélioration de la prolifération cellulaire ont été développées dans le domaine de l'ingénierie cellulaire. Notamment le document WO 01/46220 propose d'ajouter de petits peptides, notamment des oligopeptides comprenant 3 à 5 acides aminés, pour augmenter la viabilité cellulaire de cellules utilisées pour la production d'anticorps ou de protéines d'intérêt.

Dans ce document WO 01/46220, il apparaît que l'addition de glycine monomère ou de diglycine dans le milieu de culture n'augmente que très peu la prolifération des cellules d'hybridome par rapport à celui obtenu dans un milieu de culture non-supplémenté tandis que l'ajout d'un tri-, d'un tetra- ou d'un pentapeptide de glycine augmente significativement la prolifération cellulaire.

Contrairement aux résultats présentés dans ce document dans le cas de cellules hybridomes de souris, la demanderesse a constaté que, de façon surprenante, la glycylglycine ajouté dans un milieu de culture de chondrocytes conduisait à une production massive de ces cellules. En particulier, le taux de prolifération des chondrocytes dans un tel milieu est multiplié par trois, et même par sept, par rapport à celui obtenu dans un milieu de culture non-supplémenté.

Selon un premier aspect, l'invention concerne une méthode de culture pour stimuler la prolifération des cellules différenciées appartenant au lignage chondrogénique, comprenant la mise en culture desdites cellules différenciées dans un milieu de culture comprenant de la glycylglycine en quantité suffisante pour stimuler la prolifération des cellules.

En particulier, l'invention propose une méthode de culture pour stimuler la prolifération des cellules différenciées appartenant au lignage chondrogénique, dans un milieu de culture comprenant 1 à 500 mM de glycylglycine et de préférence 10 à 50 mM de glycylglycine.

Selon un deuxième aspect, l'invention propose une composition comprenant des cellules différenciées appartenant au lignage chondrogénique et un milieu de culture incorporant de la glycylglycine en quantité suffisante pour stimuler la prolifération des cellules.

Selon un troisième aspect, l'invention propose un médicament pour le traitement local de pathologies liées à l'appareil ostéo-articulaire par stimulation de la prolifération des cellules différenciées appartenant au lignage chondrogénique, ledit médicament comprenant de la glycylglycine en quantité suffisante pour stimuler la prolifération des cellules.

Enfin, selon un quatrième et dernier aspect, l'invention concerne l'utilisation de la glycylglycine pour la préparation d'un médicament destiné à traiter *in situ* les pathologies liées à l'appareil ostéo-articulaire en quantité suffisante pour stimuler la prolifération des cellules différenciées appartenant au lignage chondrogénique.

D'autres objets et avantages de l'invention apparaîtront au cours de la description qui suit, en référence aux dessins annexés dans lesquels :
- les figures 1 A et 1 B représentent des observations microscopiques de chondrocytes ostéoarthritiques humains cultivés pendant 7 jours dans des conditions identiques en absence (A) et en présence (B) de glycylglycine 20 mM.
- la figure 2 représente un test cytofluorimétrique à la PKH-26 de prolifération de chondrocytes ostéo-arthritiques humains.
- la figure 3 représente un test cytofluorimétrique à la calcéine-AM de viabilité de chondrocytes ostéoarthritiques humains cultivés pendant 7 jours en absence et en présence de glycylglycine 20 mM et adhérents au support de la culture.

Selon un premier aspect, l'invention concerne une méthode de culture pour stimuler la prolifération des cellules différenciées appartenant au lignage chondrogénique, comprenant la mise en culture desdites cellules différenciées dans un milieu de culture comprenant de la glycylglycine en quantité suffisante pour induire la prolifération des cellules.

La méthode de culture pour stimuler la prolifération des cellules différenciées est réalisée *in vitro,* par exemple après prélèvement de cartilage sur des patients.

Les cellules différenciées appartenant au lignage chondrogénique sont les cellules capables de synthétiser du collagène de type II ou de l'aggrécane à partir d'un gène endogène.

Par exemple, les cellules différenciées sont des chondrocytes extraits de tissus cartilagineux sains ou atteints d'ostéo-arthrite.

En variante, les chondrocytes sont différenciés in vitro à partir de progéniteurs tels que des cellules souches mésenchymateuses.

La méthode selon l'invention conduit à une production massive des cellules chondrogéniques, c'est-à-dire une population de cellules au moins 2 fois supérieure à la population d'origine en moins de 5 jours.

En outre, la méthode permet de conserver le phénotype des chondrocytes cultivés. En effet, la présence de glycylglycine bloque la dédifférenciation des chondrocytes en fibroblastes.

En particulier, en utilisant la méthode selon l'invention, le taux de prolifération des chondrocytes est multiplié par trois voire par sept, par rapport à celui obtenu dans un milieu de culture standard non supplémenté, pour une même période de culture.

Dans une réalisation particulière, et préalablement à la mise en culture dans un milieu comprenant de la glycylglycine, on réalise l'isolement des chondrocytes à partir de cartilage ostéoarthritique.

La méthode de culture pour stimuler la prolifération des chondrocytes comprend une étape de mise en culture des cellules des chondrocytes pendant un à sept jours, notamment moins de cinq jours, dans un milieu de base de type DMEM de Dulbecco comportant éventuellement des antibiotiques et antimycotiques (pénicilline, streptomycine, amphotéricine), de la L-glutamine et du sérum de veau foetal, le milieu étant supplémenté en glycylglycine.

Selon une réalisation, le milieu de culture comprend de 1 à 500 mM de glycylglycine, particulièrement 10 à 50 mM de glycylglycine et encore plus particulièrement 20 mM de glycylglycine.

Notamment, le milieu de culture ne contient pas de facteurs de croissance ajoutés autre que la glycylglycine. Les facteurs de croissance utilisés dans les milieux de culture étant généralement des molécules synthétiques complexes et coûteuses, un milieu exempt de telles molécules est donc une alternative particulièrement avantageuse.

La prolifération cellulaire peut être effectuée à partir de cellules saines ou malades. L'intérêt est particulièrement grand dans le cas où la prolifération est réalisée à partir de chondrocytes malades.

En effet, il a été récemment montré que des cellules de cartilage issues de tissus de patients âgés ou atteints d'ostéoarthrite étaient capables de générer de nouveaux cartilages. Cette méthode est donc particulièrement intéressante lorsque le tissu est tellement atteint que seules des cellules malades peuvent encore être prélevées. Ces cellules peuvent être extraites puis cultivées dans un milieu de culture selon l'invention.

Selon un deuxième aspect, l'invention concerne une composition comprenant des cellules différenciées appartenant au lignage chondrogénique et un milieu de culture comprenant de la glycylglycine en quantité suffisante pour stimuler la prolifération des cellules.

De manière connue, les chondrocytes cultivés et multipliés sont réimplantés dans le tissu malade du patient. Les chirurgiens qui pratiquent ce type d'interventions préparent un substitut du cartilage de forme adaptée, correspondant précisément à la lésion ostéoarthritique.

Pour cela, une méthode de réimplantation consiste à cultiver les chondrocytes dans un biomatériau poreux artificiel tel qu'une éponge de collagène ou de chitosane de manière à donner au greffon une forme adaptée à la lésion. L'éponge de collagène supportant le greffon est ensuite chirurgicalement appliquée sur la lésion. Une telle éponge est par exemple décrite dans le document RO114560.

Selon l'invention, les chondrocytes sont cultivés sur un biomatériau poreux artificiel dans un milieu de culture supplémenté en glycylglycine.

Un avantage apporté par l'invention est que la présence de glycylglycine empêche la dédifférenciation des chondrocytes en fibroblastes, phénomène qui est généralement observé au-delà de cinq jours de culture.

En outre, la prolifération étant nettement plus rapide que celle observée dans le cas de l'utilisation d'un milieu non supplémenté en glycylglycine, les cellules sont rapidement assez nombreuses et peuvent être réimplantées après seulement trois jours de culture, ce qui permet de réaliser d'importantes économies.

L'invention concerne en outre un médicament pour le traitement local de pathologies liées à l'appareil ostéo-articulaire, telles que l'ostéoarthrite, comprenant de la glycylglycine en quantité suffisante pour stimuler la prolifération des cellules. Le médicament agit par stimulation de la prolifération des cellules différenciées appartenant au lignage chondrogénique. Cette libération locale d'un facteur capable de mobiliser les chondrocytes (effet trophique local) constitue une stratégie de traitement nouvelle.

Le médicament est utilisé en médecine humaine ou vétérinaire, notamment pour soigner les lésions ostéo-arthritiques des chevaux.

Selon une première variante, le médicament se présente sous la forme d'une solution injectable de glycylglycine dissoute dans un solvant acceptable pour l'injection directe dans l'articulation ostéoarthritique. Un exemple de solvant acceptable est un sérum physiologique injectable à 0,9 % de NaCl. La solution injectable peut notamment contenir 1 à 500 mM de glycylglycine.

Un médicament injectable pour le traitement local de l'ostéoarthrite est particulièrement intéressant car il permet de reconstruire une articulation lésée tout en limitant les interventions chirurgicales intrusives.

Sous l'action de la solution de glycylglycine injectée directement dans l'articulation ostéoarthritique, les chondrocytes se multiplient et viennent remplacer la partie lésée de l'articulation.

Selon une seconde variante, le médicament consiste en un matériau implantable dans lequel est incorporé de la glycylglycine, destiné à remplacer la partie lésée du cartilage. Ce matériau, qui peut être par exemple une éponge de collagène, peut être réimplanté directement dans le cartilage sans réaliser l'étape préalable de culture des chondrocytes.

Selon cette variante, la partie lésée du cartilage est extraite et remplacée par une éponge de collagène comportant une composition de glycylglycine. L'éponge de collagène opère un relargage progressif de la glycylglycine et mobilise ainsi les chondrocytes sains présents à la périphérie de l'éponge.

Sous l'effet de la glycylglycine, les chondrocytes sains se multiplient et colonisent l'éponge de collagène jusqu'à atteindre une densité de cellules semblable à la densité du cartilage environnant.

L'avantage de cette variante est qu'elle ne nécessite qu'une seule étape de chirurgie étant donné que l'éponge de collagène peut être préparée préalablement et implantée dans l'articulation aussitôt la partie lésée retirée.

Dans une autre variante, le matériau implantable comportant de la glycylglycine comporte également des chondrocytes.

La présence de la glycylglycine dans le matériau implantable permet de maintenir la capacité des chondrocytes contenus dans l'éponge de collagène à poursuivre leur prolifération in situ après la greffe pour atteindre plus rapidement la densité cellulaire optimale du cartilage.

Selon un dernier aspect, l'invention vise donc l'utilisation de la glycylglycine pour le traitement *in situ* les pathologies liées à l'appareil ostéo-articulaire en quantité suffisante pour stimuler la prolifération des cellules différenciées appartenant au lignage chondrogénique.

L'invention sera mieux comprise à l'aide de l'exemple 1 détaillant la mise en culture des chondrocytes selon la méthode objet de l'invention, ainsi que des exemples suivants de caractérisation de la production massive des chondrocytes.

### Matériels et méthodes

Le milieu de Dulbecco DMEM a été fourni par Cambrex Bio Science (Verviers, Belgique), le sérum de veau foetal, la pénicilline, la streptomycine, l'amphotéricine B et la L-glutamine (Invitrogen), la PKH 26, la hyaluronidase, la trypsine, la collagénase de *Clostridium histolyticum* et la calcéine-AM par SIGMA (St. Louis, USA) et la N-glycylglycine par BACHEM (Villers-le-Bretonneux, France). La sénescence réplicative a été étudiée à l'aide du C12FDG (5-dodécanoulaminofluorescéine di-β-D-galactopyranoside) fourni par Molecular Probes (Eugen, USA). Le cytofluorimètre était un appareil Becton-Dickinson FACScan (SanJose, USA) équipé d'un logiciel CellQuest Pro.

### EXEMPLES

### Exemple 1 : Isolement et culture des chondrocytes

### Exemple 1.1 Isolement des chondrocytes

Le cartilage articulaire a été prélevé dans des conditions stériles sur un patient ostéoarthritique. Les chondrocytes ont été isolés selon les procédés de Green (1971) et de Kuettner et al. (1982), par dissociation enzymatique du cartilage dans le milieu DMEM de Dulbecco supplémenté avec un mélange d'antibiotiques et d'antimycotiques (pénicilline 10U/ml, streptomycine 10mg/ml, amphotéricine B 0,025mg/ml), de L-glutamine 2mM et de 10 % de sérum de veau foetal. Le cartilage a été découpé en petits fragments et incubé (37°C ; 20 min) dans le milieu DMEM précédent contenant 1mg/ml de hyaluronidase testiculaire de mouton. Les fragments ont ensuite été lavés dans du tampon PBS (Phosphate Saline Buffer) de pH 7,4, puis incubés (60 min,37°C) dans le même tampon contenant 0,25g/100mL de trypsine. Les fragments de cartilage préalablement lavés avec le tampon PBS de pH 7,4 ont été enfin dissociés par incubation (15h, 37°C) dans du milieu DMEM contenant 0,2 % de collagénase de *Clostridium histolyticum* et 10 % de sérum de veau foetal. Les cellules ont été recueillies par centrifugation (15 min, 3000g), lavées 2 fois avec le milieu DMEM complet et mises en culture dans les conditions ci-après.

### Exemple 1.2 Culture des chondrocytes

Les chondrocytes ont été cultivés sur des plaques de culture à puits en polystyrène non-coatées, à 37°C pendant 1 à 7 jours en atmosphère de CO₂ (5 %), en absence ou en présence de 20 mM de glycylglycine, en milieu DMEM contenant 10 % de sérum de veau foetal et supplémenté avec un mélange d'antibiotiques et d'antimycotiques (pénicilline 10U/ml, streptomycine 10mg/ml, amphotéricine B 0,025mg/ml) et de L-glutamine 2mM. En vue des analyses cytométriques ultérieures, les chondrocytes ont été détachés par incubation pendant 3 minutes dans une solution en tampon PBS de pH 7,4 de trypsine (0,25g/100ml) et d'EDTA (0,2g/100ml) maintenue à 37°C. Après 3 lavages avec du tampon PBS de pH 7,4, les chondrocytes, après comptage des cellules à l'aide d'une cellule de Malassez, ont été soumis aux diverses analyses par cytofluorimétrie en flux décrites ci-après.

Les figures 1A et 1 B illustrent cette production massive des chondrocytes. La figure 1A est une observation microscopique de chondrocytes cultivés dans un milieu DMEM tel que celui décrit précédemment, sans glycylglycine. La figure 1 B est une observation microscopique de chondrocytes cultivés dans un milieu DMEM identique à celui de la figure 1A, mais en présence de 20 mM de glycylglycine.

Selon les figures 1A et 1 B, le comptage des cellules permet de déterminer que le taux de prolifération des chondrocytes cultivés dans milieu DMEM supplémenté de 20 mM de glycylglycine est multiplié par trois par rapport à celui obtenu dans un milieu de culture identique non-supplémenté en glycylglycine.

### Exemple 2 : Analyse par cytométrie en flux

Les analyses ont été effectuées à l'aide d'un cytomètre FACScan équipé d'un logiciel ProCellQuest dans les conditions expérimentales suivantes : cellules en suspension dans un tampon isotonique PBS de pH 7,4, d'osmolalité 320-330 mosmol/kg, nombre de cellules analysées : 10 000.

### Exemple 2.1 Test de prolifération cellulaire

***Principe -*** Avant leur mise en culture, les chondrocytes ont été marqués irréversiblement à l'aide d'un intercalant fluorescent membranaire vital dérivé de l'acridine orange, la PKH-26, en vue de suivre par cytofluorimétrie en flux la prolifération cellulaire qui amène une diminution de la fluorescence globale des cellules.

***Mode opératoire** -* 10⁶ chondrocytes en suspension dans 1ml du « diluant C » du kit de marquage sont ajoutés à 1ml d'une solution de PKH-26 2µM dans le même diluant. Après 4 min d'incubation à température ambiante, la réaction est arrêtée par l'addition de 2ml de sérum de veau foetal puis, après une incubation d'une minute, on ajoute 4ml de milieu DMEM complet. Après centrifugation (5 min, 2000g à 25°C), le sédiment obtenu est lavé 3 fois avec 10 ml de milieu DMEM complet. Les cellules sont ensuite cultivées pendant 3 jours dans les conditions décrites dans l'exemple 1.2, détachées de leur support selon le procédé décrit plus haut, remises en suspension dans un tampon PBS de pH 7,4 et analysées par cytofluorimétrie en flux dans le mode logarithmique FL2.

La figure 2 représente les résultats de l'analyse par cytofluorométrie en flux du taux de prolifération des chondrocytes ostéoarthritiques humains marqués à la PKH-26 et cultivés en présence et en l'absence de glycylglycine 20 mM.

La courbe A illustre la fluorescence des chondrocytes marqués avant la culture, c'est à dire avant la division des cellules. La courbe B illustre la fluorescence des chondrocytes cultivés dans un milieu DMEM tel que décrit précédemment, en l'absence de glycylglycine. La courbe C illustre la fluorescence des chondrocytes cultivés dans un milieu DMEM supplémenté de 20 mM de glycylglycine.

Les cellules initialement marquées à la PKH-26 ont une forte intensité de fluorescence (courbe A). Cette intensité diminue au fur et à mesure de la division cellulaire puisque la PKH-26 se répartit alors entre les cellules issues de la division. Il apparaît clairement que l'intensité de la fluorescence des chondrocytes après la culture (courbes B et C) est inférieure à la fluorescence des cellules avant culture. Ceci confirme que des divisions cellulaires se sont bien produites et donc que la prolifération a été effective.

La comparaison entre les courbes B et C permet également d'établir que la fluorescence est encore diminuée dans le cas d'une population de chondrocytes cultivés dans le milieu DMEM décrit précédemment, supplémenté en glycylglycine. Ceci illustre bien que les divisions cellulaires ont été plus nombreuses dans ce cas que lors de la culture de chondrocytes dans un milieu DMEM non supplémenté en glycylglycine.

### Exemple 2.2 Taux de prolifération des cellules - cinétique de concentration en glycylglycine

Le tableau ci-dessous rassemble les taux de prolifération de cellules cultivées en l'absence ou en présence de différentes concentrations de glycylglycine.

Pour réaliser ce test de prolifération, 76 000 cellules sont mises en culture pendant trois jours dans les conditions de culture présentées ci-dessous.

| Concentration en Gly-Gly (mM) | Nombre de cellules obtenues (cellules/ml) | Taux de prolifération après 3 jours | Taux de prolifération / milieu de culture sans Gly-Gly |
|---|---|---|---|
| 0 | 90 000 | 18% | 1 |
| **20** | **190 000** | **150%** | **2,11** |
| 50 | 124 000 | 63 % | 1,38 |
| 100 | 145 000 | 91 % | 1,61 |
| 200 | 130 000 | 71 % | 1,44 |
| 500 | 112 000 | 47 % | 1,24 |

D'autres cultures de chondrocytes poursuivies pendant cinq jours avec une concentration en glycylglycine de 20 mM ont donné des taux de prolifération de 3 à 5 fois supérieurs au taux de prolifération observé après culture dans un milieu non-supplémenté en glycylglycine.

### Exemple 2.2 Test de la calcéine - viabilité et cytotoxicité des cellules

La viabilité des chondrocytes et la toxicité du peptide a été étudié par le procédé mis au point par Bratosin et al. (2005) et fondé sur la mesure de l'activité des estérases cellulaires par la calcéine-AM.

***Principe** -* La calcéine-AM est un ester acétique non-fluorescent de la fluorescéine qui traverse passivement les membranes des cellules viables et est transformé par les estérases cytosoliques en calcéine fluorescente qui donne un signal vert intense à 530 nm et qui n'est retenue que par les cellules possédant une membrane plasmique intacte. La disparition de la calcéine signe donc à la fois la diminution de l'activité estérasique caractéristique des cellules sénescentes, en apoptose ou soumises à l'action de substances toxiques et la fuite de ce composé hors les cellules en raison de la perméabilisation de leur membrane. Ces deux mécanismes complémentaires font de la calcéine-AM un excellent test de viabilité cellulaire et de cytotoxicité.

***Mode opératoire** -* Les chondrocytes (4x10⁵) en suspension dans 200 µl de tampon PBS de pH 7,4 sont incubés (45min à 37°C) dans l'obscurité, en présence de calcéine-AM 5µM. La suspension est ensuite additionnée de 0,5 ml de tampon PBS de pH 7,4 et analysée immédiatement par cytométrie en flux dans le mode logarithmique FL1 (Nombre de cellules comptées: 10 000).

La figure 3 représente les résultats du test à la calcéine. L'intensité de la fluorescence en échelle logarithmique est mise en abscisse et le nombre relatif de cellules est mis en ordonnée. La courbe A, réalisée au temps zéro de la culture, montre que la suspension de chondrocytes prélevés sur le cartilage ostéoarthritique, contient une proportion importante (75%) de cellules mortes. La courbe B représente le nombre de chondrocytes viables après culture dans un milieu DMEM tel que celui décrit précédemment, sans glycylglycine. La courbe C représente le nombre de chondrocytes viables après culture dans un milieu DMEM identique à celui de la courbe B, en présence de 20 mM de glycylglycine.

Les courbes B et C sont superposables, ce qui démontre que la glycylglycine ajoutée dans le milieu de culture n'est pas toxique pour les chondrocytes et ne réduit pas leur viabilité. Cette non-toxicité permet d'envisager l'utilisation in vivo de glycylglycine pour stimuler la prolifération des chondrocytes, et plus généralement des cellules différenciées du lignage chondrogénique.

## Revendications

1. Méthode de culture pour stimuler la prolifération des cellules différenciées appartenant au lignage chondrogénique, comprenant l'étape de :
- mettre en culture lesdites cellules différenciées dans un milieu de culture comprenant de la glycylglycine en quantité suffisante pour stimuler la prolifération des cellules.

2. Méthode selon la revendication 1, dans laquelle le milieu de culture comprend de 1 à 500 mM de glycylglycine.

3. Méthode selon la revendication 2, dans laquelle le milieu de culture comprend 10 à 50 mM de glycylglycine.

4. Méthode selon la revendication 3, dans laquelle le milieu de culture comprend 20 mM de glycylglycine.

5. Méthode selon l'une quelconque des revendications 1 à 4, dans laquelle les cellules mises en culture sont des chondrocytes.

6. Méthode selon la revendication 5, dans laquelle les chondrocytes sont majoritairement des chondrocytes malades.

7. Méthode selon la revendication 5 ou 6, dans laquelle les chondrocytes sont préalablement isolés par dissociation enzymatique du cartilage.

8. Méthode selon l'une des revendications 1 à 7, **caractérisée en ce que** les cellules sont mises en culture pendant une durée inférieure à cinq jours.

9. Méthode selon l'une quelconque des revendications 1 à 8, dans laquelle les cellules sont mises en culture sur un biomatériau poreux artificiel.

10. Composition comprenant des cellules différenciées appartenant au lignage chondrogénique et un milieu de culture comprenant de la glycylglycine en quantité suffisante pour stimuler la prolifération des cellules.

11. Composition selon la revendication 10, dans laquelle le milieu de culture comprend 1 à 500 mM de glycylglycine.

12. Composition selon la revendication 11, dans laquelle le milieu de culture comprend 10 à 50 mM de glycylglycine.

13. Composition selon la revendication 12, dans laquelle le milieu de culture comprend 20 mM de glycylglycine.

14. Médicament pour l'utilisation dans le traitement local de pathologies liées à l'appareil ostéo-articulaire par stimulation de la prolifération des cellules différenciées appartenant au lignage chondrogénique, **caractérisé en ce qu'**il comprend de la glycylglycine en quantité suffisante pour stimuler la prolifération des cellules.

15. Médicament selon la revendication 14, **caractérisé en ce que** la glycylglycine est dissoute dans un solvant injectable.

16. Médicament selon la revendication 15, **caractérisé en ce que** la solution injectable comprend 1 à 500 mM de glycylglycine.

17. Médicament selon la revendication 14, **caractérisé en ce que** la glycylglycine est incorporée dans un matériau implantable.

18. Médicament selon la revendication 17, **caractérisé en ce que** le matériau implantable est une éponge de collagène.

19. Utilisation de la glycylglycine pour la préparation d'un médicament destiné à traiter *in situ* les pathologies liées à l'appareil ostéo-articulaire en quantité suffisante pour stimuler la prolifération des cellules différenciées appartenant au lignage chondrogénique.

## Claims

1. Culture method for stimulating the proliferation of differentiated cells belonging to the chondrogenic lineage, comprising the step of:
- culturing said differentiated cells in a culture medium comprising glycylglycine in sufficient quantity to stimulate proliferation of the cells.

2. Method of claim 1, in which the culture medium includes 1 to 500 mM of glycylglycine.

3. Method of claim 2, in which the culture medium includes 10 to 50 mM of glycylglycine.

4. Method of claim 3, in which the culture medium includes 20 mM of glycylglycine.

5. Method as claimed in any of claims 1 to 4, in which the cultured cells are chondrocytes.

6. Method of claim 5, in which the chondrocytes are predominantly diseased chondrocytes.

7. Method as claimed in claim 5 or 6, in which the chondrocytes are isolated in advance by enzymatic dissociation of the cartilage.

8. Method as claimed in one of claims 1 to 7, **characterised in that** the cells are cultured for a period of less than five days.

9. Method as claimed in any of claims 1 to 8, in which the cells are cultured on an artificial porous biomaterial.

10. Composition comprising differentiated cells belonging to the chondrogenic lineage and a culture medium comprising glycylglycine in an amount sufficient to stimulate the proliferation of cells.

11. Composition of claim 10, in which the culture medium includes 1 to 500 mM of glycylglycine.

12. Composition of claim 11, in which the culture medium includes 10 to 50 mM of glycylglycine.

13. Composition of claim 12, in which the culture medium includes 20 mM of glycylglycine.

14. Drug for use in the local treatment of diseases related to the osteoarticular system, by stimulating the proliferation of the differentiated cells belonging to the chondrogenic lineage, **characterised in that** it includes glycylglycine in an amount sufficient to stimulate the proliferation of cells.

15. Drug of claim 14, **characterised in that** the glycylglycine is dissolved in an injectable solvent.

16. Drug of claim 15, **characterised in that** the injectable solution includes 1 to 500 mM of glycylglycine.

17. Drug of claim 14, **characterised in that** the glycylglycine is incorporated into an implantable material.

18. Drug of claim 17, **characterised in that** the implantable material is a collagen sponge.

19. Use of glycylglycine for the preparation of a drug intended for *in situ* treatment of diseases related to the osteoarticular system, in an amount sufficient to stimulate the proliferation of the differentiated cells belonging to the chondrogenic lineage.

## Patentansprüche

1. Kultivierungsverfahren zur Stimulierung der Proliferation der differenzierten Zellen, die zur chondrogenen Linie gehören, umfassend den folgenden Schritt:
- Kultivierung der differenzierten Zellen in einem Kulturmedium, umfassend Glycylglycin in einer Menge, die ausreichend ist, um die Proliferation der Zellen zu stimulieren.

2. Verfahren nach Anspruch 1, wobei das Kulturmedium zwischen 1 und 500 mM Glycylglycin umfasst.

3. Verfahren nach Anspruch 2, wobei das Kulturmedium zwischen 10 und 50 mM Glycylglycin umfasst.

4. Verfahren nach Anspruch 3, wobei das Kulturmedium 20 mM Glycylglycin umfasst.

5. Verfahren nach einem der Ansprüche 1 bis 4, wobei die kultivierten Zellen Chondrozyten sind.

6. Verfahren nach Anspruch 5, wobei die Chondrozyten zum Großteil kranke Chondrozyten sind.

7. Verfahren nach Anspruch 5 oder 6, wobei die Chondrozyten im Voraus durch enzymatische Spaltung des Knorpels isoliert werden.

8. Verfahren nach einem der Ansprüche 1 bis 7, **dadurch gekennzeichnet, dass** die Zellen während eines Zeitraums von weniger als fünf Tagen kultiviert werden.

9. Verfahren nach einem der Ansprüche 1 bis 8, wobei die Zellen auf einem künstlichen porösen Biomaterial kultiviert werden.

10. Zusammensetzung, umfassend differenzierte Zellen, die zur chondrogenen Linie gehören, und ein Kulturmedium, umfassend Glycylglycin in einer Menge, die ausreichend ist, die um die Proliferation der Zellen zu stimulieren.

11. Verfahren nach Anspruch 10, wobei das Kulturmedium zwischen 1 und 500 mM Glycylglycin umfasst.

12. Verfahren nach Anspruch 11, wobei das Kulturmedium zwischen 10 und 50 mM Glycylglycin umfasst.

13. Verfahren nach Anspruch 12, wobei das Kulturmedium 20 mM Glycylglycin umfasst.

14. Arzneimittel zur Verwendung bei der lokalen Behandlung von Pathologien, die mit dem Knochen-Gelenk-Apparat verbunden sind, durch die Stimulierung der Proliferation der differenzierten Zellen, die zur chondrogenen Linie gehören, **dadurch gekennzeichnet, dass** es Glycylglycin in einer Menge umfasst, die ausreichend ist, um die Proliferation der Zellen zu stimulieren.

15. Arzneimittel nach Anspruch 14, **dadurch gekennzeichnet, dass** das Glycylglycin in einem injizierbaren Lösemittel gelöst ist.

16. Arzneimittel nach Anspruch 15, **dadurch gekennzeichnet, dass** die injizierbare Lösung zwischen 1 und 500 mM Glycylglycin umfasst.

17. Arzneimittel nach Anspruch 14, **dadurch gekennzeichnet, dass** das Glycylglycin in ein implantierbares Material eingeschlossen ist.

18. Arzneimittel nach Anspruch 17, **dadurch gekennzeichnet, dass** das implantierbare Material ein Kollagenschwamm ist.

19. Verwendung von Glycylglycin für die Zubereitung eines Arzneimittels, das für die *in situ* Behandlung der Pathologien bestimmt ist, die mit dem Knochen-Gelenk-Apparat verbunden sind, in einer Menge, die ausreichend ist, um die Proliferation von differenzierten Zellen zu stimulieren, die zur chondrogenen Linie gehören.
